(19) Europäisches Patentamt
European Patent Office
Office européen des brevets

(11) Publication number: **0 066 773**
**B1**

(12) # EUROPEAN PATENT SPECIFICATION

(45) Date of publication of patent specification: **05.09.84**

(51) Int. Cl.$^3$: **C 07 C 103/44,** C 07 D 243/04

(21) Application number: **82104546.5**

(22) Date of filing: **25.05.82**

(54) **N-(2-(2-amino-2-phenylethyl)phenyl)-2,2-dimethyl-propanamid and derivatives and a process for preparing the same.**

(30) Priority: **28.05.81 US 267990**

(43) Date of publication of application:
**15.12.82 Bulletin 82/50**

(45) Publication of the grant of the patent:
**05.09.84 Bulletin 84/36**

(84) Designated Contracting States:
**AT BE CH DE FR GB IT LI NL SE**

(56) References cited:
**EP-A-0 009 800**

(73) Proprietor: **HOECHST-ROUSSEL PHARMACEUTICALS INCORPORATED**
**Route 202-206 North**
**Somerville New Jersey 08876 (US)**

(72) Inventor: **Everett Lee, George, Dr.**
**2 Seville Drive**
**Sommerville New Jersey 08876 (US)**
Inventor: **Bing Kin Lee, Thomas, Dr.**
**5 Latourette Road**
**Whitehouse Station New Jersey 08889 (US)**

(74) Representative: **Meyer-Dulheuer, Karl-Hermann, Dr. et al**
**HOECHST Aktiengesellschaft Zentrale Patentabteilung Postfach 80 03 20**
**D-6230 Frankfurt/Main 80 (DE)**

**EP 0 066 773 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a new intermediate of the formula

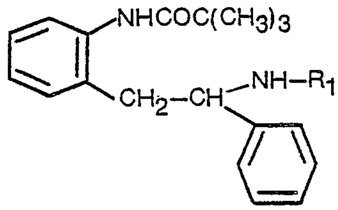

(I)

wherein $R_1$ is hydrogen or $C_1$-$C_5$-alkyl, useful for the preparation of pharmacologically active 4,5-dihydro-4-phenyl-3H-1,3-benzodiazepines, and to a method for the preparation of this intermediate, which comprises reacting an N-acylated-o-toluidine of the formula II

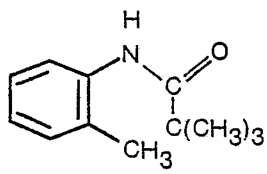

(II)

with n-alkyl lithium to provide a dilithio compound of the formula III

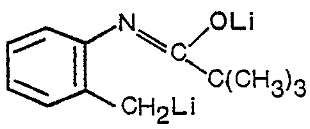

(III)

and quenching this compound with N-benzylidene amine of the formula IV

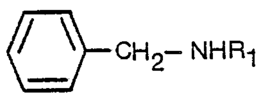

(IV)

wherein $R_1$ is hydrogen or $C_1$-$C_5$-alkyl.
Hydrolysis of the compound of the formula I yields the free base of the formula V

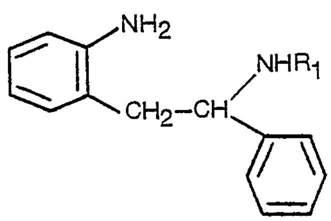

(V)

or its salt, which can be cyclized, as described in the published European Patent Application No. 0 009 800, with a compound of the formula VI.

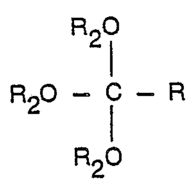

VI

wherein R is hydrogen or $C_1$-$C_5$-alkyl and $R_2$ is methyl or ethyl, to form a compound of the formula VII

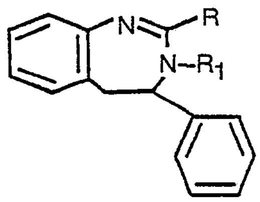

(VII)

wherein R and $R_1$ are as defined above.

This compound, its optical antipodes and physiologically acceptable salts are useful as antidepressants, analgetics and anticonvulsants. Of particular interest for these purposes are the hydrochloride salts of the 4-phenyl-1,3-benzodiazepine of formula VII.

The 4-phenyl-1,3-benzodiazepines, methods for their preparation and compounds useful as intermediates in their preparation are known from Published European Patent Application No. 0 009 800. The known methods of preparation require a relatively large number of steps, the steps are relatively complicated, the starting materials are costly and the yields of the 4-phenyl-1,3-benzodiazepines are less than desirable. Thus, there exists a need in the art for a new process for the preparation of the 4-phenyl-1,3-benzodiazepines.

This need is fulfilled by providing the process for preparing the intermediate of the formula I from which the 4-phenyl-1,3-benzodiazepines can be obtained in high yields and increased purity. The process according to the present invention utilizes more economical starting materials and a relatively small number of uncomplicated steps.

The N-acylated-o-toluidine of formula (II) is N-[(2-methyl)phenyl]2,2-dimethylpropanamide. Lithiation of aromatic compounds with an n-alkyllithium compound is exemplified in J. M. Muchowski and M. Venuti, J. Org. Chem. *45*, 4798—4801 (1980) and W. Fuhrer and H. W. Gschwend, J. Org. Chem. *44*, 1133—1136 (1979). A preferred method according to the present invention involves slowly adding a solution of n-butyllithium in a solvent therefor, such as hexane, to a solution of the N-acylated-o-toluidine in an ethereal solvent, such as diethyl ether, tetrahydrofuran, dimethoxyethane, and a hydrocarbon solvent, such as hexane. The ethereal solvent and hydrocarbon solvent should be substantially inert to the n-butyllithium to avoid adverse side reactions. The temperature during the addition can range from —70°C to 30°C, preferably about —10°C to about 30°C. The resulting mixture is aged from one-half to 5 hours, preferably about 1 to about 2 hours. The reaction is conveniently carried out at atmospheric pressure. The amount of n-butyllithium employed is up to 10% in excess of the 2 molar equivalents required for the reaction. It is important to exclude moisture from the reaction mixture. Accordingly, the reaction is conveniently conducted in an atmosphere of a substantially dry gas, such as substantially anhydrous nitrogen.

The dilithio intermediate of formula (III) is quenched with an N-benzylideneamine to provide the N-[2-(2-amino-2-pheylethyl)phenyl]-2,2-dimethylpropanamide of formula I. For instance, N-benzylidenemethylamine and a method for its preparation are disclosed by R. B. Moffett et al, Org. Syn. Coll., Vol. IV, 605—608 (1963). The temperature of addition of N-benzylidenealkylamine can range from —78°C to 35°C, preferably from about 0° to about 25°C. The mixture is aged for a period of 5 minutes to one hour. The amount of N-benzylidenealkylamine employed is from one to 2 molar equivalents based on the dilithio intermediate of formula III. The quenching is conveniently conducted at atmospheric pressure and in a substantially moisture-free, e.g., dry nitrogen, atmosphere.

The propanamide of formula I can then be hydrolyzed to provide a compound of the formula

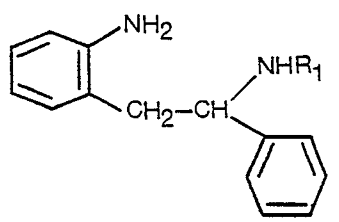

(V)

as a free base or its salt, e.g. dihydrochloride. The aromatic amine of formula V is the immediate precursor of the 4-phenyl-1,3-benzodiazepines of formula VII. Thus, it will be understood that the salts of the compound of formula V can be general be the same as the salts of the compounds of formula VII. In one method, the compound of formula I is reacted with about 2 molar equivalents of a strong mineral acid, such as hydrochloric acid, hydrobromic acid or sulfuric acid. 6N Hydrochloric acid is the acid of choice. The reaction is conveniently conducted at atmospheric pressure and at a temperature of from about 70° to the reflux temperature of the solvent employed in the reaction for a period of about 12 to about 48 hours to provide a diacid salt, which can then be recrystallized. A solvent, such as ether or an aromatic solvent, is employed to remove any side products while retaining the diacid salt in aqueous phase. If desired, the diacid salt can be bastified to provide the free base.

The aromatic amine of formula V in free base or salt form can be cyclized with a compound of the formula

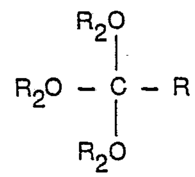

VI

wherein R is hydrogen or $C_1$-$C_5$-alkyl and $R_2$ is methyl or ethyl, to provide the 4-phenyl-1,3-benzodiazepines of formula VII. This cyclization can be carried out in the presence of an acid catalyst, such as ethanolic hydrochloric acid. Alternatively, the monoacid or diacid salt of the compound of formula V can be cyclized with a compound of formula VI in a polar solvent, such as acetonitrile or acetic acid; this makes it unnecessary to add an acid catalyst to the reaction mixture. The reaction can be conducted at a temperature of from about 23°C to the reflux temperature of the reaction mixture and at atmospheric pressure for at least about 1 hour, typically about 1 to about 8 hours. About 1 to about 5 molar equivalents of the compound of formula VI are employed.

The invention is described in greater detail in the following examples in which all parts, proportions, ratios and percentages are by weight unless otherwise indicated.

Example 1

Synthesis of trimethylacetyl chloride

A solution of trimethylacetic acid (204.3 g, 2.0 mol) in methylene chloride (400 ml) containing a catalytic amount of DMF (0.5 g) was stirred under a dry nitrogen atmosphere and treated with $SOCl_2$ (258 g, 2.06 mol). Following the addition of $SOCl_2$ (about 5 minutes) the reaction temperature dropped from 21°C to 13°C; at the same time, a vigorous evolution of HCl and $SO_2$ occurred. After 5 hours, the reaction was heated to reflux and maintained at this temperature for 2 hours. At this time, the conversion of trimethylacetic acid to trimethylacetyl chloride was quantitative. The crude product, without concentration or distillation, can be employed directly in the synthesis of N-[(2-methyl)phenyl]-2,2-dimethyl propanamide.

Example 2

Synthesis of N-[(2-methyl)phenyl]-2,2-dimethyl propanamide

(a) A biphasic solution of o-toluidine (107.2 g, 1.0 mol) in methylene chloride (500 ml) and water (150 ml) containing sodium carbonate (69 g, 0.65 mol) was treated with trimethylacetyl chloride (120.6 g, 1.0 mol). The rate of addition of trimethylacetyl chloride was adjusted so as to maintain the reaction at gentle reflux. After 45 minutes the addition was complete. The organic layer was separated, washed with water, and concentrated *in vacuo*. The crude N-[(2-methyl)phenyl)-2,2-dimethylpropanamide was slurried in 2% aqueous HCl, filtered and washed with $H_2O$ until the filtrate was neutral. After drying *in vacuo* (50°C, 20 mm), N-[(2-methyl)phenyl]-2,2-dimethylpropanamide (178 g, 0.93 mol) was obtained in 93% yield. (mp 109—110°C).

(b) A biphasic solution of o-toluidine (214.4 g, 2.0 mol) in methylene chloride (200 ml) and water (250 ml) containing sodium carbonate (117 g, 1.1 mol) was treated with trimethylacetyl chloride (about 2.0 mol in methylene chloride from Example 1). The addition of trimethylacetyl chloride was complete after 50 minutes; the temperature ranged between 37—50°C during the addition. The warm organic phase was separated and the aqueous phase was extracted with methylene chloride (2 × 100 ml).

The combined methylene chloride solution was washed with 1N HCl (2 × 100 ml), $H_2O$ (3 × 200 ml), 10% NaCl (100 ml), and concentrated *in vacuo* (25°C at 30 mm) to give free flowing crystalline N-[(2-methyl)phenyl]-2,2-dimethylpropanamide. Final drying at (60°C, 30 mm, 24 hrs) have N-[(2-methyl)phenyl]-2,2-dimethylpropanamide (379 g, 1.98 mol) in 99% yield. The melting point of the product was 108—111°C. This product can be used directly in Example 4 (b) without recrystallization.

Example 3

Synthesis of N-Benzylidenemethylamine

(a) Anhydrous monomethylamine (about 1—1.5 eq.) was introduced below the surface of a stirred solution of benzaldehyde (1062 g, 10.0 mol) in toluene (2000 ml) cooled to 0°C. The rate of addition of monomethylamine was adjusted so as to maintain the reaction temperature between 25—30°C; after 45 minutes the addition of monomethylamine was terminated. The organic phase was separated and concentrated *in vacuo*. Distillation of the residual oil afforded N-benzylidenemethylamine (1047 g, 8.79 mol) in 88% yield. The product has a boiling point of 79°C at 20 mm. [This N-benzylidinemethylamine starting material is reported by R. B. Moffett et al, *Org. Syn. Coll.* Vol. IV, 605—608 (1963)].

(b) Anhydrous monomethylamine (about 1—15 eq.) was bubbled into a stirred solution of benzaldehyde (531 g, 5.0 mol) in toluene (1000 ml) at 0°C. The rate of addition of monomethylamine was adjusted so as to maintain the reaction temperature between 20—25°C. The progress of the reaction was evaluated by GC [using a 6′ — 2 mm, 3% OV—101 (silicone polymer) on 80/100 mesh Chromosorb W Column.] After 30 minutes, monomethylamine addition was stopped; at 50 minutes

into the reaction the conversion of benzaldehyde to N-benzylidinemethylamine was 99%. At this point, the aqueous phase was separated and the toluene phase was concentrated *in vacuo* (50—60°C at 30 mm) to give a colorless to pale yellow oil (657 g). This crude oil was shown by NMR and GC to contain 76% N-benzylidinemethylamine and 24% toluene. The conversion to N-benzylidinemethylamine was greater than 99.7% by GC. The calculated yield of N-benzylidinemethylamine was 84%. This crude product can be used in subsequent reactions without distillation.

Example 4
Synthesis of N-[2-(2-methylamino-2-phenyl-ethyl)-phenyl]-2,2-dimethylpropanamide

(a) A stirred solution of N-[(2-methyl)phenyl]-2,2-dimethylpropanamide (95.6 g, 0.5 mol) in THF (500 ml, dried over 4 A molecular sieves) was cooled to 0°C and treated with 1.6 M n-butyllithium in hexane (630 ml, 1.0 mol). The addition of n-butyllithium was complete after 45 minutes. During the addition, the temperature of the mixture was maintained below 10°C with external cooling. The resultant dianion solution was aged (about 1—2 hrs) at 0°C until the homogeneous orange solution became a white heterogeneous slurry. The dianion was then quenched with N-benzylidenemethylamine (63.7 g, 0.6 mol) and aged for 30 minutes at 15—25°C. The reaction mixture was diluted with ether (200 ml), treated with crushed ice (200 g), and stirred for 15 minutes. The organic phase was separated, washed with saturated sodium chloride, dried over anhydrous magnesium sulfate and concentrated *in vacuo*. The residual oil was crystallized from hexane (400 ml) at 5°C to give N-[2-(2-methylamino-2-phenyl-ethyl)phenyl]-2,2-dimethylpropanamide (121.2 g, 0.39 mol) in 78% yield. The product had a melting point of 85—86°C.

(b) A heterogeneous mixture of N-[(2-methyl)phenyl]-2,2-dimethylpropanamide (95.6 g, 0.5 mol) and THF (200 ml) was stirred under a dry nitrogen atmosphere at 0—10°C. The first equivalent of 1.6M n-butyllithium in hexane (305 ml) was rapidly added (about 5 min) causing the temperature to increase to 20°C and at the same time causing a complete dissolution of starting material. (The end point for the first equivalent can be judged gravimetrically or by observance of a distinctive color change produced upon formation of trace amounts of the dianion). A second equivalent of 1.6M n-butyllithium in hexane (305 cc) was added and the dianion solution aged at 0—5°C for 1—2 hours. N-benzylidenemethylamine (89 cc) of a 76% solution in toluene containing 0.6 mol was added causing the heterogeneous mixture to become homogeneous and biphasic (the resultant monoanion is immiscible when the ratio of the THF:hexane is decreased as in this Example). The biphasic solution was stirred for 30 minutes at 0—5°C then treated with $H_2O$ (300 ml). The organic phase was separated, dried over anhydrous sodium sulfate, filtered, and concentrated *in vacuo* (30 mm) at 45°C to give a crystalline product. Recrystallization from cold hexane (300 ml) gave a N-[2-(2-methylamino-2-phenylethyl)phenyl]-2,2-dimethylpropanamide (118 g, 0.38 mol) in 76% yield with a melting point of 85—86°C.

Example 5
Synthesis of N-methyl-2-amino-$\alpha$-phenylphenethylamine dihydrochloride

(a) N-[2-(2-methylamino-2-phenylethyl)phenyl]-2,2-dimethylpropanamide (62 g, 0.2 mol) was dissolved in 6 N HCl (124 g) and stirred under a nitrogen atmosphere at 100°C for 24 hours. The warm reaction mixture (about 35—40°C) was extracted with toluene (2 × 100 ml) to effect recovery of trimethylacetic acid. The aqueous phase was dried by azeotropic distillation with toluene (200 ml) using a Dean-Stark phase separator. The product was collected by filtration, slurried in hot 2-propanol (200 ml), refiltered and dried *in vacuo* (30 mm) at 45°C for 12 hours to give N-methyl-2-amino-$\alpha$-phenylphenethylamine dihydrochloride (58.7 g, 0.196 mol) in 98% yield. The product had a melting point of 251—253°C.

(b) N-[2-(2-methylamino-2-phenylethyl)phenyl]-2,2-dimethylpropanamide (155 g, 0.5 mol) was dissolved in 6 N HCl (310 g) and stirred under a nitrogen atmosphere at 100°C for 28 hours. The reaction mixture was cooled to 23°C and toluene (200 ml) was added. Stirring was continued until the product had crystallized from the aqueous phase. The product was collected by filtration, washed with toluene (2 × 50 ml) and dried *in vacuo* (30 mm) at 60°C for 60 hours to give N-methyl-2-amino-$\alpha$-phenylphenethyl-amine dihydrochloride (139 g, 93% yield). The melting point of the product was 252—254°C. The organic phase of the filtrate was concentrated *in vacuo* to give a 58% recovery of trimethylacetic acid. The aqueous phase of the filtrate afforded a second crop of N-methyl-2-amino-$\alpha$-phenylphenethylamine dihydrochloride (9.3 g, 6%). The total yield of product was 99%.

Example 6
Synthesis of 4,5-dihydro-2,3-dimethyl-4-phenyl-3H-1,3-benzodiazepine hydrochloride

A heterogeneous mixture of N-methyl-2-amino-$\alpha$-phenylphenethylamine dihydrochloride (150 g, 0.5 mol) in acetonitrile (500 ml) was treated with triethylorthoacetate (202 ml, 1.1 mol) and heated to 70°C for 2 hours with stirring under a dry nitrogen atmosphere. The reaction mixture was filtered, concentrated *in vacuo*, and the residual solid recrystallized from 2-propanol at −10°C to give 2,3-dimethyl-4-phenyl-3H-1,3-benzodiazepine hydrochloride (118 g, 0.413 mol) in 83% yield. The product had a melting point of 239.5—241°C.

Example 7

Synthesis of 4,5-dihydro-2,3-dimethyl-4-phenyl-3H-1,3-benzodiazepine (free base)

A heterogeneous mixture of the dihydrochloride salt of N-ethyl-2-amino-$\alpha$-phenylphenethylamine (9 g, 0.03 mol) in acetonitrile (36 cc dried over 4 Å molecular sieves) was treated with triethylorthoacetate (9.73 g, 11 cc, 0.06 mol) and heated to 70°C with stirring under a dry nitrogen atmosphere. At 50°C, (after about 15 minutes of heating) the reaction mixture became homogeneous. The reaction mixture was concentrated *in vacuo* and partitioned between 100 ml toluene and 50 ml 5% NaOH. The toluene phase was washed with 10% NaCl, dried over sodium sulfate, filtered and concentrated *in vacuo* to give a light yellow-brown solid product, which was shown by GC to be 96.7% 2,3-dimethyl-4-phenyl-3H-1,3-benzodiazepine. This crude product free base was recrystallized from 15 ml 2-propanol at 82°C, diluted with 30 ml hexane and dried at 45°C (30 mm for 12 hours). 4.64 g (0.0185 mol) of product with a melting point of 144.5°—145.4°C were obtained at a yield of 61.8%. A second crop was recrystallized from 5 ml 2-propanol, washed with 3 ml hexane and dried at 40°C (30 mm for 12 hours), 1.92 g (0.0077 mol) of product with a melting point of 143.5°—144.5°C were obtained at a yield of 25.6%. The mother liquor (0.64 g) from the first crop contained 0.24 g of product representing a yield of 3.2%.

Example 8

Conversion of 4,5-dihydro-2,3-dimethyl-4-phenyl-3H-1,3-benzodiazepine (free base) to hydrochloride salt

1.9 g (0.0759 mol) of the free base 2,3-dimethyl-4-phenyl-3H-1,3-benzodiazepine obtained in Example 7 was dissolved in 2-propanol and treated at 5—10°C with an excess of 2-propanol saturated with anhydrous HCl. The monohydrochloride salt of the 4,5-dihydro-2,3-dimethyl-4-phenyl-3H-1,3-benzodiazepine was recrystallized from solution and recovered by filtration. The monohydrochloride salt had a melting point of 241—242°C. The salt was dried (60°C, 30 mm, 24 hours) to yield 1.50 g of a white crystalline powder.

**Claims**

1. Compound of the formula I:

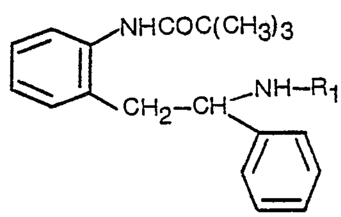

(I)

wherein $R_1$ is hydrogen or $C_1$-$C_5$-alkyl.

2. A process for preparing a compound of the formula I

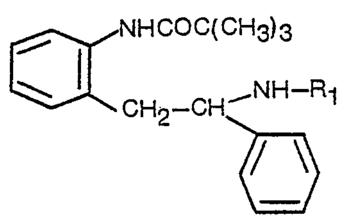

(I)

wherein $R_1$ is hydrogen or $C_1$-$C_5$-alkyl, which comprises reacting an N-acylated-o-toluidin of the formula II

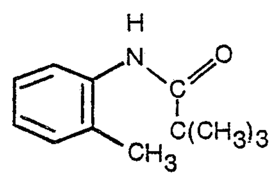

(II)

with n-alkyllithium to provide a dilithio intermediate of the formula III

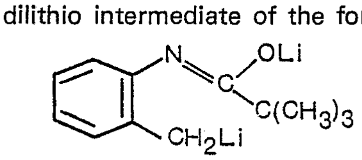

(III)

and quenching this compound with an N-benzylidene amine of the formula IV

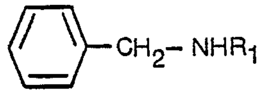

(IV)

wherein $R_1$ is hydrogen or $C_1$-$C_5$-alkyl.

3. Process according to claim 2 comprising reacting the N-acylated-o-toluidine with n-alkyllithium in solution in inert ethereal and hydrocarbon solvents and in a substantially anhydrous atmosphere.

4. Process according to claim 3 wherein the ethereal solvent is selected from a group consisting of diethyl ether, tetrahydrofuran and dimethoxyethane, and the hydrocarbon solvent is hexane.

5. Process according to claim 2 comprising reacting the N-acylated-o-toluidine with n-alkyllithium at −70°C to 30°C for 0.5 to 5 hours.

6. Process according to claim 3 wherein said reaction is conducted at −10°C to 30°C for 1 to 2 hours.

7. Process according to claim 3 wherein a solution of the n-butyllithium is added to the N-acylated-o-toluidine.

8. Process according to claim 2 in which the quenching is conducted at −78°C to 35°C for 5 minutes to 1 hours in a substantially anhydrous atmosphere.

9. Process according to claim 8 in which the quenching is conducted at about 0°C to about 25°C with 1 to 2 molar equivalents of the N-benzylidenealkylamine based on the dilithio intermediate.

10. A process for preparing 4,5-dihydro-4-phenyl-3H-1,3-benzodiazepine or a pharmaceutically acceptable salt thereof, of the formula VII.

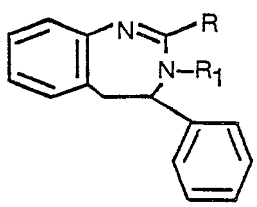

(VII)

wherein R and $R_1$ are the same or different, and each is hydrogen or $C_1$-$C_5$-alkyl, which comprises hydrolyzing a compound of the formula I

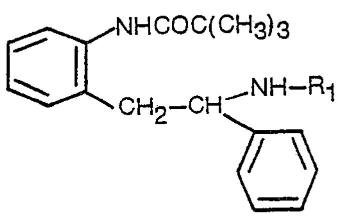

(I)

wherein $R_1$ is as defined above, to form a compound of the formula V

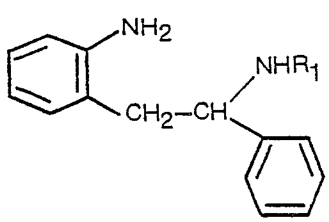

(V)

or its salt, and cyclizing it with a compound of the formula VI

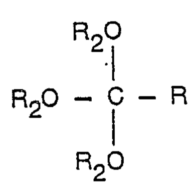

VI

wherein R is as defined above and $R_2$ is methyl or ethyl.

**Patentansprüche**

1. Verbindung der Formel I

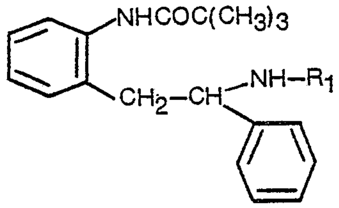

(I)

worin $R_1$ Wasserstoff oder $C_1$-$C_5$-Alkyl bedeutet.

2. Verfahren zur Herstellung einer Verbindung der Formel I

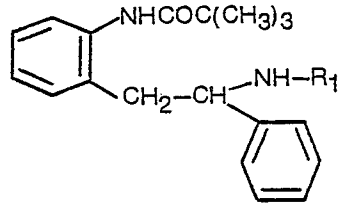

(I)

worin $R_1$ Wasserstoff oder $C_1$-$C_5$-Alkyl bedeutet, dadurch gekennzeichnet, daß ein N-acryliertes o-Toluidin der Formel II

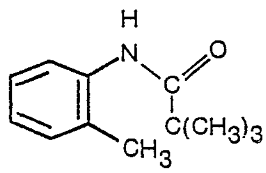

(II)

mit N-Alkyl-Lithium zu einer Dilithioverbindung der Formel III

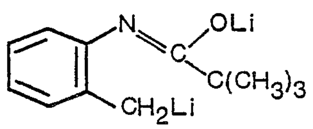

(III)

umgesetzt wird und letztere mit einem N-Benzylidenamin der Formel IV

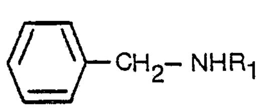

(IV)

worin $R_1$ Wasserstoff oder $C_1$-$C_5$-Alkyl bedeutet, umgesetzt wird.

3. Verfahren nach Anspruch 2, dadurch gekennzeichnet, daß das N-acylierte o-Toluidin mit n-Alkyllithium in inerten ätherischen Lösungsmitteln oder in Kohlenwasserstoffen gelöst in weitgehend wasserfreier Atmosphäre umgesetzt wird.

4. Verfahren nach Anspruch 3, dadurch gekennzeichnet, daß man als ätherisches Lösungsmittel Diethyläther, Tetrahydrofuran oder Dimethoxyethan und als Kohlenwasserstoff Hexan verwendet.

5. Verfahren nach Anspruch 2, dadurch gekennzeichnet, daß das N-acrylierte o-Toluidin mit n-Alkyllithium bei —70°C bis 30°C 0,5—5 Stunden umgesetzt wird.

6. Verfahren nach Anspruch 3, dadurch gekennzeichnet, daß die Reaktion bei einer Temperatur von —10°C bis 30°C während 1—2 Stunden abläuft.

7. Verfahren nach Anspruch 3, dadurch gekennzeichnet, daß man das N-acylierte o-Toluidin mit einer Lösung aus n-Butyllithium versetzt.

8. Verfahren nach Anspruch 2, dadurch gekennzeichnet, daß die Reaktion mit der Verbindung der Formel IV bei —78°C bis 35°C während eines Zeitraumes von 5 Minuten bis 1 Stunde in weitgehend wasserfreier Atmosphäre abläuft.

9. Verfahren nach Anspruch 8, dadurch gekennzeichnet, daß die Reaktion mit der Verbindung der Formel IV bei einer Temperatur von ca. 0°C bis ca. 25°C mit 1—2 Moläquivalenten N-Benzylidenalkylamin, bezogen auf die Dilithiozwischenverbindung, abläuft.

10. Verfahren zur Herstellung von 4,5-Dihydro-4-phenyl-3H-1,3-benzodiazepin oder eines seiner pharmazeutisch verträglichen Salze, der Formel VII

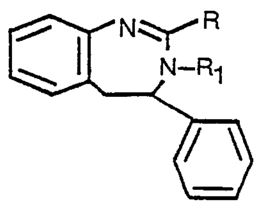

(VII)

dadurch gekennzeichnet, daß R und $R_1$ gleich oder verschieden sind und je Wasserstoff oder $C_1$-$C_5$-Alkyl bedeuten, dadurch gekennzeichnet, daß eine Verbindung der Formel I

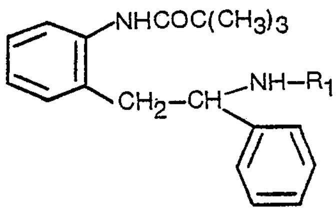

(I)

worin $R_1$ die obige Bedeutung hat, zu einer Verbindung der Formel V

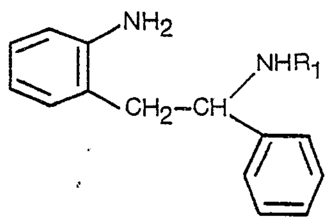

(V)

oder einem ihrer Salze hydrolysiert wird und letztere mit einer Verbindung der Formel VI

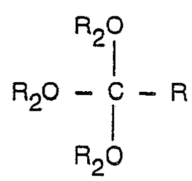

VI

worin R die obige Bedeutung hat und $R_2$ Methyl oder Ethyl bedeutet, cyclisiert wird.

**Revendications**

1. Un composé de formule I

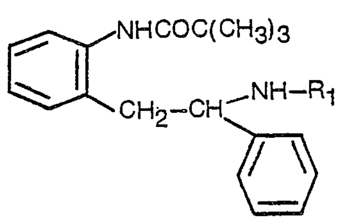

(I)

dans laquelle $R_1$ est l'hydrogène ou un groupe alcoyle en $C_1$-$C_5$.

2. Un procédé de préparation d'un composé de formule I

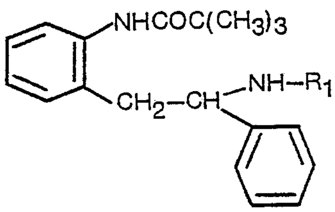

(I)

dans laquelle $R_1$ est l'hydrogène ou un groupe alcoyle en $C_1$-$C_5$, caractérisé en ce qu'on fait réagir une o-toluidine N-acylée de formule II

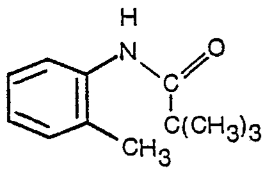

(II)

avec un n-alcoyl lithium pour obtenir un intermédiaire dilithio de formule III

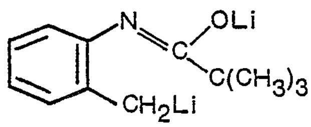

(III)

et on fait réagir ce composé avec une N-benzylidène amine de formule IV

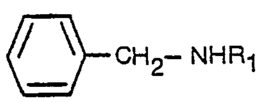

(IV)

dans laquelle $R_1$ est l'hydrogène ou un groupe alcoyle en $C_1$-$C_5$.

3. Procédé selon la revendication 2, caractérisé en ce qu'on fait réagir l'o-toluidine N-acylée avec le n-alcoyl lithium en solution dans des solvants éthérés et hydrocarbonés, inertes et sous une atmosphère pratiquement anhydre.

4. Procédé selon la revendication 3, caractérisé en ce que le solvant éthéré est choisi parmi l'éther diéthylique, le tétrahydrofurane et le diméthoxyéthane, et le solvant hydrocarboné est l'hexane.

5. Procédé selon la revendication 2, caractérisé en ce qu'on fait réagir l'o-toluidine N-acylée avec le n-alcoyl lithium entre −70 et +30°C pendant de 0,5 à 3 heures.

6. Procédé selon la revendication 3, caractérisé en ce que la réaction est effectuée entre −10 et +30°C pendant de 1 à 2 heures.

7. Procédé selon la revendication 3, caractérisé en ce qu'on ajoute une solution du n-butyl lithium à l'o-toluidine N-acylée.

8. Procédé selon la revendication 2, caractérisé en ce que la réaction avec le composé de formule IV est effectuée entre −78 et +35°C pendant de 5 minutes à 1 heure sous une atmosphère pratiquement anhydre.

9. Procédé selon la revendication 8, caractérisé en ce que la réaction avec le composé de formule IV est effectuée entre environ 0 et 25°C avec de 1 à 2 équivalents molaires de la N-benzylidène alcoylamine, par rapport à l'intermédiaire dilithio.

10. Procédé de préparation de la 4,5-dihydro-4-phényl-3H-1,3-benzodiazépine ou d'un de ses sels pharmaceutiquement acceptables, de formule VII

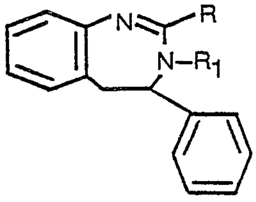

(VII)

dans laquelle R et $R_1$ sont identiques ou différents et sont chacun l'hydrogène ou un groupe alcoyle en $C_1$-$C_5$, caractérisé en ce qu'on hydrolyse un composé de formule I

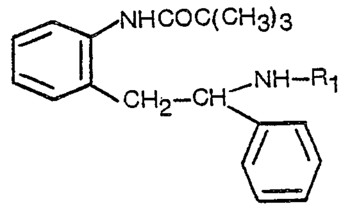

(I)

dans laquelle $R_1$ est tel que défini ci-dessus, pour former un composé de formule V

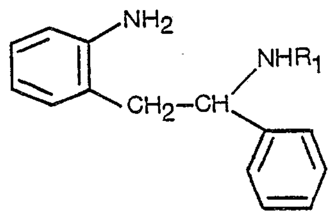

(V)

ou son sel, et on cyclise ce composé avec un composé de formule VI

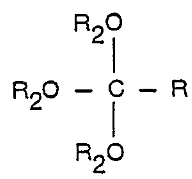

VI

dans laquelle R est tel que défini ci-dessus et $R_2$ est un groupe méthyle ou éthyle.